(19) Europäisches Patentamt
European Patent Office
Office européen des brevets

(11) EP 1 776 946 A2

(12) **DEMANDE DE BREVET EUROPEEN**

(43) Date de publication:
**25.04.2007 Bulletin 2007/17**

(51) Int Cl.:
***A61K 8/81*** *(2006.01)* ***A61Q 19/08*** *(2006.01)*

(21) Numéro de dépôt: **06301066.4**

(22) Date de dépôt: **19.10.2006**

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **21.10.2005 FR 0553215**

(71) Demandeur: **L'ORÉAL**
**75008 Paris (FR)**

(72) Inventeurs:
- **Schultze, Xavier**
**77340, PONTAULT-COMBAULT (FR)**
- **Vicic, Marco**
**94360, BRY SUR MARNE (FR)**

(74) Mandataire: **Le Coupanec, Pascale A.M.P.**
**Nony & Associés,**
**3 rue de Penthièvre**
**75008 Paris (FR)**

(54) **Composition cosmétique contenant un polymère statistique à chaîne principale linéaire de nature éthylénique**

(57) La présente invention concerne une composition cosmétique comprenant, dans un milieu physiologiquement acceptable, au moins un copolymère statistique à chaîne principale linéaire de nature éthylénique, dans laquelle ledit copolymère :
(i) présente une masse moléculaire en poids comprise entre 15 000 et 600 000 g/mol, et consiste en :
(ii) au moins 70 % en poids d'« unités monomères » issues de monomères dont les homopolymères sont hydrophobes et présentent une température de transition vitreuse supérieure à 40 °C,
(iii) au moins une « unité monomère » issue d'un monomère hydrophile ionique au moins partiellement neutralisée,
(iv) 0 à 25 % en poids d'« unités monomères » issues de monomères additionnels dont les homopolymères présentent une température de transition vitreuse inférieure à 40 °C choisis parmi :
- les acrylates de formule :

$$CH_2 = CHCOOR_{12}$$

où $R_{12}$ représente un groupe alkyle en $C_1$ à $C_{12}$ linéaire ou ramifié à l'exception du groupe tcrtiobutylc, dans lequel se trouvc(nt) éventuellement intcrcalé(s) un ou plusieurs hétéroatomes identiques ou non, ledit groupe alkyle pouvant en outre être éventuellement substitué par un ou plusieurs substituants identiques ou non choisis parmi les groupes hydroxyle et les atomes d'halogène, et
- les méthacrylates de formule :

$$CH_2 = C(CH_3)COOR_{14}$$

où $R_{14}$ représente un groupe alkyle en $C_4$ à $C_{12}$ linéaire ou un groupe alkyle en $C_5$ à $C_{12}$ ramifié, et
(v) présente une température globale de transition vitreuse supérieure ou égale à 45 °C.

EP 1 776 946 A2

## Description

La présente invention se rapporte à une composition antirides contenant au moins un copolymère statistique à chaîne principale linéaire de nature éthylénique, et à l'utilisation de ce copolymère comme agent tenseur dans une composition cosmétique, destinées notamment au traitement, à la diminution, à l'effacement et/ou au lissage des rides et ridules de la peau de l'être humain.

Au cours du processus de vieillissement, il apparaît différents signes sur la peau, très caractéristiques de ce vieillissement, se traduisant notamment par une modification de la structure et des fonctions cutanées. Les principaux signes cliniques du vieillissement cutané sont notamment l'apparition de ridules et de rides profondes, en augmentation avec l'âge. On constate en particulier une désorganisation du « grain » de la peau, c'est-à-dire que le micro-relief est moins régulier et présente un caractère anisotrope.

Il est connu de traiter ces signes du vieillissement en utilisant des compositions cosmétiques ou dermatologiques contenant des actifs capables de lutter contre le vieillissement, tels que les $\alpha$-hydroxy-acides, les $\beta$-hydroxy-acides et les rétinoïdes. Ces actifs agissent sur les rides en éliminant les cellules mortes de la peau et en accélérant le processus de renouvellement cellulaire. Toutefois, ces actifs présentent l'inconvénient de n'être efficaces pour le traitement des rides qu'après un certain temps d'application. Or, on cherche de plus en plus à obtenir un effet immédiat des actifs utilisés, conduisant rapidement à un lissage des rides et ridules et à la disparition des marques de fatigue.

La présente invention a justement pour objet l'utilisation d'un copolymère particulier permettant d'obtenir immédiatement cet effet.

Il est connu d'utiliser certains agents tenseurs d'origine naturelle, tels que les protéines et hydrolysats de protéines d'origine végétale, comme cela est notamment décrit dans WO 98/29091 ou encore un polysaccharide végétal et de la caséine hydrolysée comme actifs tenseurs comme cela est notamment décrit dans WO 96/19180. Toutefois, l'utilisation de substances d'origine naturelle est limitée pour des raisons sanitaires et d'effet tenseur minime.

Il est par ailleurs connu d'utiliser des matériaux inorganiques telles que la silice colloïdale et les argiles comme agents tenseurs. Les performances atteintes à ce titre sont très importantes. Toutefois, l'effet tenseur de ces matériaux inorganiques s'annule en émulsion en présence de glycérine.

D'autres compositions à effet tenseur de l'art antérieur utilisent des polymères synthétiques.

On connaît ainsi de WO 98/29092 une composition à effet tenseur comprenant une dispersion aqueuse d'un système polymérique contenant au moins un polymère d'origine synthétique présentant un poids moléculaire supérieur à 670 000 g/mol, choisi parmi différents types de polyuréthannes, les polyurées, les polymères ou copolymères acryliques, les polymères d'acide isophtalique sulfoné et leurs mélanges. Le toucher cosmétique de ces compositions n'est cependant pas toujours satisfaisant.

On connaît également de EP 1 038 519 l'utilisation de certains polymères siliconés spécifiques pour jouer le rôle d'agents à effet tenseur, ou encore de WO 00/30595 des copolymères contenant en poids de 20 à 90 % d'un lactame vinylique, de 1 à 55 % d'acide carboxylique polymérisable et de 1 à 25 % d'un monomère hydrophobe tel qu'un acrylate ou méthacrylate d'alkyle en $C_{10}$ à $C_{24}$ permettant de fournir des gels mous utilisables dans des compositions cosmétiques antirides.

Enfin, le document FR 2 843 025 décrit l'utilisation de réseaux interpénétrés de polymères en tant qu'agent pour lisser les rides et ridules et/ou retendre la peau et le document FR 2 822 676 décrit une composition cosmétique filmogène comprenant au moins, à titre d'agent filmogène, un copolymère acrylique dans une forte teneur, à savoir comprise entre 20 et 50 % en poids par rapport au poids de la composition.

Toutefois, il subsiste toujours le besoin de composés offrant un effet tenseur immédiat, suffisant et durable, sans risque pour le consommateur.

En outre, il existe un besoin de matériaux polymériques synthétiques présentant un effet tenseur fort tout en pouvant être formulés en présence de glycérine.

Enfin, il existe un besoin de matériaux polymériques synthétiques véhiculables dans un milieu aqueux et présentant une rigidité très importante après application sur la peau et après évaporation des matières volatiles.

La présente invention a donc pour objet une composition cosmétique comprenant, dans un milieu physiologiquement acceptable, au moins un copolymère statistique à chaîne principale linéaire de nature éthylénique, dans laquelle ledit copolymère :

(i) présente une masse moléculaire en poids comprise entre 15 000 et 600 000 g/mol, et consiste en :
(ii) au moins 70 % en poids d'« unités monomères » issues de monomères dont les homopolymères sont hydrophobes et présentent une température de transition vitreuse supérieure à 40 °C,
(iii) au moins une « unité monomère » issue d'un monomère hydrophile ionique au moins partiellement neutralisée, et
(iv) 0 à 25 % en poids d'« unités monomères » issues de monomères additionnels dont les homopolymères présentent une température de transition vitreuse inférieure à 40 °C choisis parmi :

- les acrylates de formule :

$$CH_2 = CHCOOR_{12}$$

où $R_{12}$ représente un groupe alkyle en $C_1$ à $C_{12}$ linéaire ou ramifié (à l'exception du groupe tertiobutyle), dans lequel se trouve(nt) éventuellement intercalé(s) un ou plusieurs hétéroatomes identiques ou non, ledit groupe alkyle pouvant en outre être éventuellement substitué par un ou plusieurs substituants identiques ou non choisis parmi les groupes hydroxyle et les atomes d'halogène, et

- les méthacrylates de formule :

$$CH_2 = C(CH_3)COOR_{14}$$

où $R_{14}$ représente un groupe alkyle en $C_4$ à $C_{12}$ linéaire ou un groupe alkyle en $C_5$ à $C_{12}$ ramifié, et
(v) présente une température globale de transition vitreuse supérieure ou égale à 45 °C.

L'invention a également pour objet l'utilisation comme agent tenseur, dans une composition cosmétique, d'au moins un copolymère statistique à chaîne principale linéaire de nature éthylénique, ledit copolymère étant tel que défini précédemment.

Dans le cadre de cette description et des revendications annexées, on entend par « agent tenseur » des composés susceptibles d'avoir un effet tenseur apparent, c'est-à-dire de lisser la peau et réduire, voire faire disparaître, de façon immédiate les rides et les ridules.

Plus précisément, on peut considérer qu'une composition présente un effet tenseur lorsque dans le test exposé à l'exemple 10-1, la valeur de l'effet tenseur ET est supérieure à 60 %.

La composition utilisée dans la présente invention contient, en plus du copolymère précité, un milieu physiologiquement acceptable, c'est-à-dire compatible avec la peau et ses phanères, les muqueuses et les semi-muqueuses.

La présente invention se rapporte également à un procédé de traitement cosmétique d'une peau vieillie, notamment ridée, comprenant l'application sur ladite peau d'au moins une composition comprenant au moins un copolymère statistique à chaîne principale linéaire de nature éthylénique tel que défini précédemment, en une quantité efficace pour estomper les rides par un effet tenseur.

Le terme « de nature éthylénique » dans la cadre de la présente invention qualifie les polymères comportant une chaîne principale comportant uniquement des unités monomères,

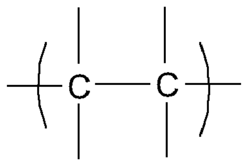

les chaînes latérales pouvant être constituées d'atomes de carbone, d'oxygène, d'azote, d'hydrogène, de soufre et/ou de phosphore.

On note en particulier que la présence d'atomes de silicium dans la chaîne latérale est exclue de la portée de l'invention.

Le terme « unité monomère » désigne l'unité constitutive la plus grande de la structure d'une macromolécule formée à partir d'une même molécule de monomère.

Le terme « alkyle », dans le cadre de la présente invention, signifie une chaîne hydrocarbonée linéaire ou ramifiée, saturée ou insaturée, cyclique ou non cyclique. Parmi les groupes alkyle convenant à la mise en oeuvre de l'invention, on peut notamment citer le groupe méthyle, éthyle, isopropyle, n-propyle, n-butyle, t-butyle, -$CH_2$-t-butyle, pentyle, n-hexyle, cyclopropyle, cyclopentyle, cyclohexyle, cyclohexylméthyle, heptyle, octyle, nonyle, décyle, norbornyle et adamantyle.

Le terme « aryle », dans le cadre de la présente invention, signifie un système mono- ou bicyclique possédant un ou deux noyaux aromatiques. Parmi les groupes aryle, on peut citer le phényle, le napthyle, le tétrahydronaphtyle, l'indanyle.

Le terme « aralkyle » signifie un groupe aryle lié à un groupe alkyle, tel que le groupe benzyle.

Le terme « groupe hétérocyclique » signifie un cycle de 4 à 12 chaînons contenant un ou plusieurs hétéroatomes identiques ou non choisis parmi O, N, S ou P. Ledit groupe hétérocyclique peut comprendre ou non des doubles liaisons.

Ce terme comprend en outre les groupes bicycliques dans lesquels un hétérocycle à 3, 4, 5 ou 6 chaînons est fusionné avec un groupe phényle ou avec un cycloalkyle tel que le cyclohexane ou encore avec un autre hétérocycle.

Parmi ces groupes hétérocycliques, on peut citer l'indolyle, le quinolyle, l'isoquinolyle, le tétrahydroquinolyl, le benzofuryle et le benzothiènyle. Le terme « groupe hétérocyclique » recouvre notamment le pyrrolyle, le pyrrolinyle, le pyrrolidinyle, le pyrazolyle, le pyrazolinyle, le pyrazolidinyle, l'imidazolyle, l'imidazolinyle, l'imidazolidinyle, le pyridyle, le pipéridinyle, le pyrazinyle, le pipérazinyle, le pyrimidinyle, le pyridazinyle, l'oxazolyle, l'oxazolidinyle, l'isoxazolyle, l'isoxazolidinyle, le morpholinyle, le thiazolyle, le thiazolidinyle, l'isothiazolyle, l'isothiazolidinyle, l'indolyle, le quinolinyle, l'isoquinolinyle, le benzimidazolyle, le benzothiazolyle, le benzoxazolyle, le furyle et le thiényle.

Le terme « hétérocyclylalkyle » signifie un groupe hétérocyclique lié à un groupe alkyle.

Les atomes d'halogène comprennent le chlore, le brome, l'iode et le fluor.

Les hétéroatomes, sauf mention contraire, comprennent les atomes d'oxygène, d'azote, de soufre et de phosphore.

Le terme « compris entre ... et... » signifie que les bornes sont également décrites.

## COPOLYMERES

Le copolymère présent dans la composition selon la présente invention est un copolymère statistique à chaîne principale linéaire de nature éthylénique.

La composition peut également comprendre un mélange de tels copolymères.

Le copolymère utile dans le cadre de la présente invention présente une masse moléculaire en poids comprise entre 15 000 et 600 000 g/mol. La masse moléculaire est de préférence comprise entre 20 000 et 200 000 g/mol, et va de façon encore plus préférée de 55 000 à 200 000 g/mol. Par « masse moléculaire en poids », on entend la masse moléculaire Mp au pic de la courbe de distribution.

Par ailleurs, sa température globale de transition vitreuse est supérieure ou égale à 45 °C, notamment allant de 45 °C à 300 °C.

On entend par « température de transition vitreuse », dont l'abréviation est « Tg », la température au-dessous de laquelle le polymère est rigide. Lorsque la température augmente, le polymère passe par un état de transition qui permet aux chaînes macromoléculaires de glisser les unes par rapport aux autres et le polymère se ramollit.

On utilise le terme « température globale de transition vitreuse » pour signifier que le copolymère peut comporter des monomères distincts, dont les homopolymères respectifs peuvent présenter des températures de transition vitreuse différentes et que c'est le copolymère en tant que tel qui présente ladite température globale de transition vitreuse.

Ainsi, on préfère dans le cadre de la présente invention utiliser des copolymères présentant une température globale de transition vitreuse supérieure à 60 °C, notamment supérieure à 60 °C et inférieure à 300 °C.

Dans le cadre de la présente invention, le protocole de mesure de la température de transition vitreuse des copolymères ou des homopolymères formés par les monomères utiles pour la préparation des copolymères utilise une caractérisation par DSC (Differential Scanning Calorimetry) et est détaillé ci-après :

Les transitions du film (transitions vitreuses, fusions...) sont étudiées par DSC sur la base de 2 cycles de chauffage / refroidissement à 10 °C/min entre -140 °C et 130 °C (environ 2 heures). Les mesures sont réalisées sous balayage d'azote et en utilisant des coupelles hermétiques pour ne pas modifier la composition du film, par vaporisation du solvant, au cours de l'étude DSC. Le film de polymère est préparé par séchage de la solution aqueuse directement déposée (40 μl) dans les creusets d'analyse thermique. Le séchage de la solution se fait dans des conditions contrôlées pendant 48 heures à température ambiante et à 50+/-5 % d'humidité relative.

- Appareil : DSC 2920 de TA Instruments
- Gaz de purge : Azote Alphagaz 2 à 50 ml/min
- Creuset : coupelle Inox sertie de 50 μl de Perkin Elmer
- Calibration en énergie et température : Fusion de l'Indium
- Prise d'essai : conditionnée par le séchage (environ 10 mg)
- Traitements thermiques :

    1. CO : Refroidissement de +25 °C à - 140 °C à 10 °C/min
    2. CO bis : Equilibrage à - 140 °C
    3. H1 : Chauffage de - 140 °C à + 130 °C à 10 °C/min
    4. C1 : Refroidissement de + 130 °C à- 140 °C à 10 °C/min
    5. C 1 bis : Equilibrage à - 140 °C
    6. H2 : Chauffage de - 140 °C à + 130 °C à 10 °C/min

Deux échantillons sont étudiés pour chaque produit.

Le copolymère se présente de préférence en dispersion dans un liquide polaire, ledit liquide polaire étant de préférence de l'eau.

Lorsque le copolymère se présente en dispersion aqueuse, sa teneur dans ladite dispersion peut aller de 0,1 à 30 % en poids et de préférence de 5 à 20 % en poids, exprimé en matière sèche, par rapport au poids total de ladite dispersion. Comme cela est illustré dans les exemples, le copolymère peut typiquement être dispersé à 7 % en poids dans de l'eau et donner lieu à un effet tenseur supérieur à 70 % dans le test de rétraction exposé dans les exemples.

La structure du copolymère est détaillée dans la description qui va suivre.

Le copolymère comprend au moins 70 % en poids par rapport à son poids total d'« unités monomères » issues de monomères dont les homopolymères présentent une température de transition vitreuse supérieure à 40 °C. Ces « unités monomères » peuvent être de la même nature ou de nature différente. Autrement dit, le copolymère peut comprendre un seul et même type d'« unités monomères » dont l'homopolymère présente une température de transition vitreuse supérieure à 40 °C ou bien des « unités monomères » de natures distinctes, étant entendu que les monomères correspondants sont tous conformes à l'exigence rappelée ci-dessus concernant la température de transition vitreuse.

En particulier, le copolymère selon l'invention comprend plus de 72 %, notamment plus de 75 %, de préférence plus de 80 % en poids par rapport à son poids total d'« unités monomères » issues de monomères dont les homopolymères présentent une température de transition vitreuse supérieure à 40 °C, et notamment jusqu'à 95 % en poids.

Le copolymère comprend en outre au moins une « unité monomère » issue d'un monomère hydrophile ionique, ainsi que de 0 à 25 % en poids d'« unités monomères » issues de monomères additionnels dont les homopolymères présentent une température de transition vitreuse inférieure à 40 °C.

*Monomères hydrophobes dont les homopolymères présentent une température de transition vitreuse supérieure à 40 °C*

Par « monomère hydrophobe », on entend au sens de la présente invention un monomère dont l'homopolymère est insoluble dans l'eau à une concentration supérieure à 5 % en poids, à 25 °C, et qui ne forme pas non plus dans l'eau, dans ces conditions, une dispersion ou suspension stable de fines particules, généralement sphériques, ayant une taille moyenne de particule inférieure à 1 µm, et plus généralement comprise entre 5 et 400 nm, voire entre 10 et 250 nm, telle que mesurée par diffusion de lumière.

Parmi les monomères dont sont issues les « unités monomères » hydrophobes citées précédemment, on préfère ceux dont les homopolymères présentent une température de transition vitreuse supérieure ou égale à 60 °C, et notamment inférieure ou égale à 300 °C.

Les monomères utiles pour la préparation des copolymères compris dans les compositions selon la présente invention et dont les homopolymères ont des températures de transition vitreuse supérieures à 40 °C sont, de préférence, choisis parmi les composés vinyliques, les acrylates, les méthacrylates et les (méth)acrylamides, et en particulier parmi les monomères suivants :

- les composés vinyliques de formule :

$$CH_2 = CHR_1,$$

où $R_1$ est un groupe :

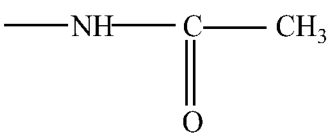 , un groupe 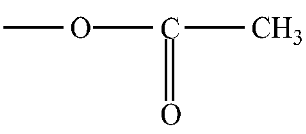 ;

un groupe cycloalkyle en $C_3$ à $C_8$ un groupe aryle en $C_6$ à $C_{20}$ ; un groupe aralkyle en $C_7$ à $C_{30}$ (groupe alkyle en $C_1$ à $C_4$) ; un groupe hétérocyclique ; un groupe hétérocyclylalkyle (alkyle en $C_1$ à $C_4$) tel qu'un groupe furfuryle ; lesdits groupes cycloalkyle, aryle, aralkyle, hétérocyclique, ou hétérocyclylalkyle pouvant être éventuellement substitués par un ou plusieurs substituants identiques ou non choisis parmi les groupes hydroxyle, les atomes d'halogène, et les groupes alkyles en $C_1$ à $C_4$ linéaires ou ramifiés dans lesquels se trouve(nt) éventuellement intercalé(s) un ou plusieurs hétéroatomes identiques ou non, et lesdits groupes alkyle pouvant, en outre, être éventuellement substitués par un ou plusieurs substituants choisis parmi les groupes hydroxyle et les atomes d'halogène.

Selon une variante préférée, $R_1$ est un groupe

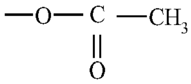
,

un groupe cycloalkyle en $C_3$ à $C_8$ ou un groupe aryle en $C_6$ à $C_{20}$. Des exemples particulièrement préférés de monomères vinyliques dont les homopolymères présentent une température de transition vitreuse supérieure à 40 °C sont le vinylcyclohexane, le styrène et l'acétate de vinyle.

- Les acrylates de formule :

$$CH_2 = CHCOOR_2$$

où $R_2$ est un groupe tertiobutyle ; un groupe cycloalkyle en $C_3$ à $C_8$ éventuellement ponté par un groupe alkylène en $C_1$ à $C_4$ éventuellement substitué par un plusieurs groupes alkyles en $C_1$ à $C_4$ ; un groupe aryle en $C_6$ à $C_{20}$ ; un groupe aralkyle en $C_7$ à $C_{30}$ (groupe alkyle en $C_1$ à $C_4$) ; un groupe hétérocyclique ; un groupe hétérocyclylalkyle (alkyle en $C_1$ à $C_4$); lesdits groupes cycloalkyle, aryle, aralkyle, hétérocyclique ou hétérocyclylalkyle pouvant être éventuellement substitués par un ou plusieurs substituants identiques ou non choisis parmi les groupes hydroxyle, les atomes d'halogène, et les groupes alkyle en $C_1$ à $C_4$ linéaires ou ramifié dans lesquels se trouve(nt) éventuellement intercalé(s) un ou plusieurs hétéroatomes identiques ou non, lesdits groupes alkyle pouvant, en outre, être éventuellement substitués par un ou plusieurs substituants identiques ou non choisis parmi les groupes hydroxyle et les atomes d'halogène.

Selon une variante préférée, $R_2$ est un groupe tertiobutyle, un groupe cycloalkyle en $C_3$ à $C_8$ éventuellement ponté par un groupe alkylène en $C_1$ à $C_4$, ou un groupe aralkyle en $C_7$ à $C_{30}$ (groupe alkyle en $C_1$ à $C_4$).

Des exemples particulièrement préférés d'acrylates dont les homopolymères présentent une température de transition vitreuse supérieure à 40 °C sont l'acrylate de benzyle, l'acrylate de cyclohexyle, l'acrylate de tertiobutyle, l'acrylate d'isobornyle et l'acrylate de norbornyle.

- Les méthacrylates de formule :

$$CH_2 = C(CH_3)COOR_3$$

où $R_3$ est un groupe isobutyle ou tertiobutyle ou un groupe alkyle en $C_1$ à $C_3$, linéaire ou ramifié, tel qu'un groupe méthyle, éthyle, propyle ou isobutyle, ces groupes alkyle pouvant en outre être éventuellement substitués par un ou plusieurs substituants identiques ou non choisis parmi les groupes hydroxyle et les atomes d'halogène ; un groupe cycloalkyle en $C_3$ à $C_8$ éventuellement ponté par un groupe alkylène en $C_1$ à $C_4$ éventuellement substitué par un plusieurs groupes alkyle en $C_1$ à $C_4$ ; un groupe aryle en $C_6$ à $C_{20}$ ; un groupe aralkyle en $C_7$ à $C_{30}$ (groupe alkyle en $C_1$ à $C_4$) ; un groupe hétérocyclique ; un groupe hétérocyclylalkyle (alkyle en $C_1$ à $C_4$) ; lesdits groupes cycloalkyle, aryle, aralkyle, ou hétérocyclique ou hétérocyclylalkyle pouvant être éventuellement substitués par un ou plusieurs substituants identiques ou non choisis parmi les groupes hydroxyle, les atomes d'halogène, et les groupes alkyle en $C_1$ à $C_4$ linéaires ou ramifié dans lequel se trouve(nt) éventuellement intercalé(s) un ou plusieurs hétéroatomes identiques ou non, lesdits groupes alkyle pouvant, en outre, être éventuellement substitués par un ou plusieurs substituants identiques ou non choisis parmi les groupes hydroxyle et les atomes d'halogène.

Selon une variante préférée, $R_3$ est un groupe isobutyle, tertiobutyle, un groupe alkyle en $C_1$ à $C_3$ linéaire ou ramifié, un groupe cycloalkyle en $C_3$ à $C_8$ éventuellement ponté par un groupe alkylène en $C_1$ à $C_4$ ou un groupe aralkyle en $C_7$ à $C_{30}$ (groupe alkyle en $C_1$ à $C_4$).

Des exemples particulièrement préférés de méthacrylates dont les homopolymères présentent une température de transition vitreuse supérieure à 40 °C sont le méthacrylate de méthyle, d'éthyle, d'isobutyle, de cyclohexyle, de benzyle, de tertiobutyle, d'isobornyle et de norbornyle.

- Les (méth)acrylamides de formule :

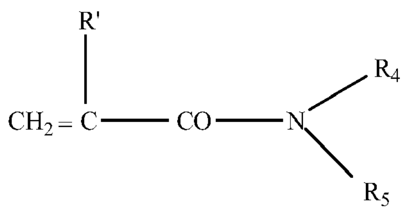

où R' désigne H ou $-CH_3$, et où $R_4$ et $R_5$ identiques ou différents représentent chacun un atome d'hydrogène ou un groupe alkyle linéaire ou ramifié en $C_4$ à $C_{12}$, étant entendu que $R_4$ et $R_5$ ne peuvent représenter simultanément un atome d'hydrogène.

Des exemples particulièrement préférés de monomères (méth)acrylamide dont les homopolymères présentent une température de transition vitreuse supérieure à 40 °C sont le N-butylacrylamide, le N-t-butylacrylamide et le N,N-dibutylacrylamide.

Selon un mode de réalisation préféré de l'invention, les monomères dont les homopolymères présentent une température de transition vitreuse supérieure à 40 °C sont choisis parmi le styrène, l'acrylate de benzyle, l'acrylate de cycloalkyle en $C_3$ à $C_8$ éventuellement ponté par un groupe alkylène en $C_1$ à $C_4$, l'acrylate de tertiobutyle, le méthacrylate de $(C_1-C_3)$alkyle, le méthacrylate de tertiobutyle, le méthacrylate de benzyle et le méthacrylate de cycloalkyle en $C_3$ à $C_8$ éventuellement ponté par un groupe alkylène en $C_1$ à $C_4$.

Les monomères dont les homopolymères présentent une température de transition vitreuse supérieure à 40 °C préférés sont l'acrylate de benzyle, l'acrylate de cyclohexyle, l'acrylate de tertiobutyle, l'acrylate d'isobornyle et l'acrylate de norbornyle, le méthacrylate de méthyle, d'éthyle, d'isobutyle, de cyclohexyle, de benzyle, de tertiobutyle, d'isobornyle et de norbornyle et le styrène. Plus préférentiellement, ils sont choisis parmi le méthacrylate de méthyle et le méthacrylate de cyclohexyle.

*Monomère hydrophile ionique*

Le copolymère présent dans la composition selon la présente invention comprend en outre au moins une « unité monomère » issue d'un monomère hydrophile ionique au moins partiellement neutralisée.

Ce monomère hydrophile ionique peut notamment être choisi parmi les monomères hydrophiles anioniques, les monomères hydrophiles cationiques, les monomères amphotères et leurs mélanges.

Typiquement, le copolymère peut contenir entre 5 et 30 % en poids d'« unités monomères » issues de monomères hydrophiles ioniques par rapport à son poids total. Plus particulièrement, le copolymère peut contenir entre 5 et 25 % en poids, notamment entre 5 et 20 % en poids, de préférence entre 5 et 18 % en poids, d'« unités monomères » issues de monomères hydrophiles ioniques. Le copolymère peut par exemple contenir entre 10 et 25 %, notamment entre 10 et 20 % en poids, de préférence entre 10 et 18 % en poids d'« unités monomères » issues de monomères hydrophiles ioniques par rapport à son poids total.

Selon un mode préféré de l'invention le copolymère contient entre 5 et 25 % en poids d'« unités monomères » issues de monomères hydrophiles ioniques ; étant entendu que lorsque le copolymère contient un ou des monomère(s) additionnel(s) dont le ou les homopolymère(s) (respectifs) présente(nt) une température de transition vitreuse inférieure à 40 °C, l'un au moins de ces monomère(s) additionnel(s) étant l'acrylate d'éthylhexyle, le copolymère contient alors entre 5 et 18 % en poids d'« unités monomères » issues de monomères hydrophiles ioniques.

Par « partiellement neutralisée », on entend au sens de la présente invention, une « unité monomère » présentant un taux de neutralisation non nul, et notamment supérieur ou égal à 50 %.

Avantageusement, l'ensemble des « unités monomères » présentent un taux de neutralisation supérieur ou égal à 50 %, de préférence supérieur ou égal à 70 %, en particulier d'au moins 90 %, voire de 100 %.

Le taux de neutralisation peut être défini comme étant le rapport du nombre de fonctions ioniques neutralisées sur le nombre de fonctions ionisées initiales, c'est à dire avant neutralisation.

De façon avantageuse, le taux de neutralisation de l'ensemble des « unités monomères » sera ajusté de sorte à permettre une mise en dispersion dans l'eau des copolymères selon l'invention.

Parmi les monomères hydrophiles anioniques, sont compris les monomères de formule (I) :

$$CH_2=CR_6\,(Z)_n\,(R_7)_m\,Y \qquad (I)$$

où Z prend l'une des significations suivantes : C(=O)O, C(=O)NH, O, O(C=O) (acétates),
n est égal à 0 ou 1, de préférence égal à 0,
m est égal à 0 ou 1, de préférence égal à 0,

$R_6$ est un atome d'hydrogène, un groupe $CH_3$, ou un groupe ($C_3$-$C_{30}$)alkyle,
$R_7$ est choisi parmi les groupements alkylènes linéaires saturés ou non et/ou branchés et/ou cycliques (aromatiques ou non) en $C_1$ à $C_{30}$, incluant ou non un ou plusieurs hétéroatomes, et
Y est choisi parmi les groupes suivants : -COOH, $-SO_3H$, $-OSO_3H$, $-OP(OH)_2$, $-OPO(OH)_2$

Préférentiellement $R_6$ est un atome d'hydrogène, un groupe $CH_3$ ou $C_2H_5$, de préférence $CH_3$.

$R_7$ est par exemple choisi parmi les groupes alkylène en $C_1$ à $C_{30}$, phénylène, benzylène, $-(CH_2-CH=CH)-$ et -(CHOH)-. De préférence $R_7$ est choisi parmi les groupes alkylène en $C_1$ à $C_6$ linéaires ramifiés ou cycliques, les groupes phénylène et benzylène.

Selon un mode de réalisation particulier, $R_6$ est un atome d'hydrogène, un groupe $CH_3$ ou un $C_2H_5$, de préférence un groupe $CH_3$ et $R_7$ est choisi parmi les groupes alkylène en $C_1$ à $C_{30}$, phénylène, benzylène, $((CH_2-CH=CH)-$ et (CHOH).

On peut citer notamment parmi les monomères hydrophiles anioniques :

- les monomères à insaturation(s) éthylénique(s) comprenant au moins une fonction acide carboxylique (COOH), phosphonique ($PO_3H_2$) ou sulfonique ($SO_3H$), comme par exemple l'acide acrylique, l'acide méthacrylique, le (méth) acrylate de 2-carboxyéthyle, l'acide vinylbenzoïque, l'acide acrylamidoglycolique $CH_2=CHCONHCH(OH)CO_2H$, l'acide maléique, l'acide acrylamidopropanesulfonique, l'acide acrylamido 2-méthylpropanesulfonique, l'acide styrène sulfonique, l'acide vinylsulfonique, le méthacrylate ou l'acrylate d'acide propyl-3-sulfonique ($CH_2=C(CH_3)CO_2(CH_2)_3SO_3H$), le méthacrylate ou l'acrylate d'acide éthyl-2-sulfonique ($CH_2=C(CH_3)CO_2(CH_2)_2SO_3H$) et la vinylméthylsulfone, l'acide vinylphosphonique ($CH_2=CH-PO(OH)_2$), le méthacrylate d'acide éthyl-2-phosphonique ($CH_2=C(CH_3)COOCH_2CH_2OP(O)(OH)_2$),
- les anhydrides carboxyliques porteurs d'une liaison vinylique tels que l'anhydride maléique,
- les diacides tels que l'acide crotonique, l'acide itaconique, l'acide fumarique, l'acide maléique,
- ainsi que leurs mélanges.

De préférence, le monomère hydrophile anionique est l'acide (méth)acrylique.

La neutralisation des groupes anioniques peut être effectuée par une base minérale, telle que LiOH, NaOH, KOH, $Ca(OH)_2$, $NH_4OH$ ou $Zn(OH)_2$; ou par une base organique telle qu'une alkylamine primaire, secondaire ou tertiaire, notamment la triéthylamine ou la butylamine. Cette alkylamine primaire, secondaire ou tertiaire peut comporter un ou plusieurs atomes d'azote et/ou d'oxygène et peut donc comporter par exemple une ou plusieurs fonctions alcool; on peut notamment citer l'amino-2-méthyl-2-propanol, la triéthanolamine et la diméthylamino-2-propanol. On peut encore citer la lysine ou la 3-(diméthylamino)propylamine.

Parmi les monomères hydrophiles cationiques, sont compris les monomères de formule (II) :

$$CH_2=CR_8\ (Z)_n\ (R_9)_m\ X \qquad (II)$$

- où Z prend l'une des significations suivantes : C(=O)O, C(=O)NH, O, O(C=O),
- n est égal à 0 ou 1,
- m est égal à 0 ou 1,
- $R_8$ est un atome d'hydrogène, un groupe $CH_3$, ou un groupe ($C_2$-$C_{30}$)alkyle,
- $R_9$ est choisi parmi les groupements alkylènes linéaires saturés ou non et/ou branchés et/ou cycliques, aromatiques ou non, en $C_1$ à $C_{30}$, incluant ou non un ou plusieurs hétéroatomes,
- X est un groupe défini par $N-R_{10}R_{11}$, ou bien X constitue un cycle, aromatique ou non, comportant un groupement amine tertiaire cationisable, inclus dans le cyle ou comme substituant, ou peut représenter un hétérocycle aromatique, ou non, contenant un azote tertiaire, cationisable inclus dans le cycle ou comme substituant ou bien X prend l'une des significations suivantes : guanidino, amidino, ou phosphino, et
- $R_{10}$ et $R_{11}$ sont choisis indépendamment parmi un atome d'hydrogène et des groupements alkyles linéaires et/ou branchés et/ou cycliques,aromatiques ou non, en $C_1$ à $C_{16}$, comportant ou non des hétéroatomes ou bien $R_{10}$ et $R_{11}$ forment avec l'atome d'azote auquel ils sont rattachés, un hétérocycle en $C_4$ à $C_{30}$, fusionné ou non, et éventuellement substitué par un ou plusieurs radicaux identiques ou non choisis parmi les groupes alkyle en $C_1$ à $C_4$, le groupe hydroxyle, un groupe alcoxy en $C_1$ à $C_4$, et un atome d'halogène.

$R_9$ est de préférence choisi parmi les groupes alkyle en $C_1$ à $C_{30}$, phénylène, benzylène, $-(CH_2-CH=CH)-$ et -(CHOH)-.

Avantageusement, $R_9$ est choisi parmi les groupes alkylène en $C_1$ à $C_6$ linéaires ramifiés ou cycliques, les groupes phénylène et benzylène.

De préférence $R_{10}$, $R_{11}$ sont choisis parmi un atome d'hydrogène et les groupes $CH_3$, $C_2H_5$, $C_3H_7$ et $C_4H_9$.

Des exemples d'hétérocycles convenant à la signification de X dans sa deuxième alternative, sont des pyridines, indolyle, isoindolinyle, imidazolyle, imidazolinyle, pipéridinyle, pyrazolynyle, pyrazolyle, quinoline, pyrazolinyle, pyridinyle, pipérazinyle, pyrrolidinyle, quinidinyle, thiazolinyle, morpholine et leurs mélanges.

Comme exemples de monomères hydrophiles cationiques, on peut citer les monomères à insaturation(s) éthylénique(s) comprenant au moins une fonction amine tertiaire ou secondaire ou primaire. On peut notamment citer :

- l'allylamine, allylpyridine ;
- les (méth)acrylates d'aminoalkyles, tels que les (méth)acrylates de [N,N-di($C_1$-$C_4$)alkylamino]($C_1$-$C_6$)alkyle ou les (méth)acrylates de [N-($C_1$-$C_4$)alkylamino]($C_1$-$C_6$)alkyle et notamment le (méth)acrylate de N,N diméthylaminoéthyle, le (méth)acrylate de N,N-diéthylaminoéthyle, le (méth)acrylate de 2-aminoéthyle, le (méth)acrylate de 2-(N-terbutylamino)éthyle;
- les (méth)acrylamides d'aminoalkyles, tels que les (méth)acrylamides de [N,N-di($C_1$-$C_4$)alkylamino]($C_1$-$C_6$)alkyle N,N-di($C_1$-$C_4$)alkylamino)alkyle ou les (méth)acrylamides [N-($C_1$-$C_4$)alkylamino]($C_1$-$C_6$)alkyle, et notamment le (méth)acrylamide de N,N-diméthylaminopropyle, le (méth)acrylamide de N,N-diméthylaminoéthyle et le (méth) acrylamide de 3-aminopropyle;
- la vinylamine, le 2-(diéthylamino)éthylstyrène;
- N-vinylimidazole, N-vinyl-2-méthylimidazole, N-vinylcarbazole ;
- ainsi que leurs mélanges, et leurs formes quaternisées.

Pour neutraliser les monomères cationiques, on peut utiliser les sels d'acides minéraux, tels que l'acide sulfurique, l'acide chlorhydrique, l'acide bromhydrique, l'acide iodhydrique, l'acide phosphorique et l'acide borique.

On peut également citer les sels d'acides organiques, qui peuvent comporter un ou plusieurs groupes acide carboxylique, sulfonique ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.

Les groupes amines tertiaires peuvent être quaternisés :

- par des composés à halogène mobile, notamment des halogénures d'alkyles tels que des chlorures ou des bromures d'alkyles en $C_1$ à $C_{12}$, et par exemple le bromure de méthyle ou le chlorure d'éthyle,
- par des composés à halogène mobile, comportant des fonctions acides carboxyliques ou sulfoniques (éventuellement salifiées). Ainsi sont obtenus des monomères hydrophiles amphotères (encore dénommés bétaines).

Les quaternisants peuvent par exemple être le chloroacétate de sodium ou des sulfones cycliques, par exemple la propanesulfone.

Cette réaction de quaternisation et (ou) de salification peut avoir lieu sur le polymère déjà synthétisé ou sur le monomère de départ avant de procéder à la polymérisation.

Comme exemples de monomères amphotères, on peut citer les carboxybétaïnes ou sulfobétaïnes éthyléniques telles que la N,N-diméthyl-N-(2-méthacryloyloxyéthyl)-N-(3-sulfopropyl) ammonium bétaine ; la N,N-diméthyl-N-(3-méthacrylamidopropyl)-N-(3-sulfopropyl)ammonium bétaine et la 1-(3-sulfopropyl)-2-vinylpyridinium bétaine.

Le monomère hydrophile cationique préféré est le N,N' diméthylaminoéthyl (méth)acrylate,

Le monomère hydrophile préféré est l'acide (méth)acrylique.

*Copolymères préférés*

Selon un mode de réalisation particulièrement préféré, le copolymère est choisi parmi :

- Les copolymères de méthacrylate de méthyle / acide méthacrylique ; les copolymères de méthacrylate de méthyle / acide acrylique, lesdits copolymères contenant entre 70 et 90 % en poids de méthacrylate de méthyle ;
- Les copolymères de méthacrylate d'éthyle/ acide méthacrylique ; les copolymères de méthacrylate d'éthyle / acide acrylique, lesdits copolymères contenant entre 70 et 90 % en poids de méthacrylate d'éthyle ;
- Les copolymères de méthacrylate d'isobutyle/ acide méthacrylique ; les copolymères de méthacrylate d'isobutyle / acide acrylique, lesdits copolymères contenant entre 70 et 90 % en poids de méthacrylate d'isobutyle ;
- Les copolymères de méthacrylate de benzyle / acide méthacrylique ; les copolymères de méthacrylate de benzyle / acide acrylique, lesdits copolymères contenant entre 70 et 90 % en poids de méthacrylate de benzyle ;
- Les copolymères d'acrylate de benzyle / acide méthacrylique ; les copolymères d'acrylate de benzyle / acide acrylique, lesdits copolymères contenant entre 70 et 90 % en poids d'acrylate de benzyle ;
- Les copolymères de méthacrylate de cyclohexyle / acide méthacrylique ; les copolymères de méthacrylate de cyclohexyle / acide acrylique, lesdits copolymères contenant entre 70 et 90 % en poids de méthacrylate de cyclohexyle ;

- Les copolymères d'acrylate de cyclohexyle / acide méthacrylique ; les copolymères d'acrylate de cyclohexyle / acide acrylique, lesdits copolymères contenant entre 70 et 90 % en poids d'acrylate de cyclohexyle ;
- Les copolymères de méthacrylate de tertio-butyle/ acide méthacrylique ; les copolymères de méthacrylate de tertio-butyle / acide acrylique, lesdits copolymères contenant entre 70 et 90 % en poids de méthacrylate de tertio-butyle ;
- Les copolymères d'acrylate de tertio-butyle/ acide méthacrylique ; les copolymères d'acrylate de tertio-butyle / acide acrylique, lesdits copolymères contenant entre 70 et 90 % en poids d'acrylate de tertio-butyle ;
- Les copolymères de méthacrylate d'isobornyle/ acide méthacrylique ; les copolymères de méthacrylate d'isobornyle / acide acrylique, lesdits copolymères contenant entre 70 et 90 % en poids de méthacrylate d'isobornyle ;
- Les copolymères d'acrylate d'isobornyle / acide méthacrylique ; les copolymères d'acrylate d'isobornyle / acide acrylique, lesdits copolymères contenant entre 70 et 90 % en poids d'acrylate d'isobornyle ;
- Les copolymères de méthacrylate de norbornyle/ acide méthacrylique ; les copolymères de méthacrylate de norbornyle / acide acrylique, lesdits copolymères contenant entre 70 et 90 % en poids de méthacrylate de norbornyle ;
- Les copolymères d'acrylate de norbornyle / acide méthacrylique ; les copolymères d'acrylate de norbornyle / acide acrylique, lesdits copolymères contenant entre 70 et 90 % en poids d'acrylate de norbornyle ; et
- Les copolymères de styrène / acide méthacrylique ; les copolymères de styrène / acide acrylique, lesdits copolymères contenant entre 70 et 90 % en poids de styrène.

*Monomère de Tg < 40 °C*

Selon un mode de réalisation particulier, les copolymères comportant en outre au moins une « unité monomère » issue d'un monomère dont l'homopolymère correspondant présente une température de transition vitreuse inférieure à 40 °C (et notamment supérieure à -100 °C) font également partie de l'invention pour autant que la température globale de transition vitreuse dudit copolymère reste bien supérieure ou égale à 45 °C.

Selon un autre mode de réalisation particulier, les monomères additionnels, dont les homopolymères présentent une température de transition vitreuse inférieure à 40 °C, dits « monomères de Tg < 40 °C » diffèrent des monomères hydrophobes et des monomères hydrophiles ioniques décrits précédemment.

La teneur en monomères dont l'homopolymère présente une température de transition vitreuse inférieure à 40 °C est ajustée de sorte que l'effet tenseur du copolymère ne se trouve pas affecté.

Leur teneur dans le copolymère est avantageusement inférieure ou égale à 25 % en poids, par rapport au poids total du copolymère, (notamment de 0 à 25 % en poids, et de préférence de 5 % à 25 % en poids) de manière encore plus préférée inférieure
ou égale à 10 % en poids (notamment de 0 à 10 % en poids, et de préférence de 5 à 10 % en poids).

Les monomères dont les homopolymères ont des températures de transition vitreuse inférieure à 40 °C sont choisis parmi les monomères suivants :

- les hydrocarbures éthyléniques en $C_2$ à $C_{10}$, tels que l'éthylène, l'isoprène et le butadiène.
- les acrylates de formule :

$$CH_2 = CHCOOR_{12}$$

où $R_{12}$ représente :

  - un groupe alkyle en $C_1$ à $C_{12}$ linéaire ou ramifié (à l'exception du groupe tertiobutyle), dans lequel se trouve (nt) éventuellement intercalé(s) un ou plusieurs hétéroatomes identiques ou non, ledit groupe alkyle pouvant en outre être éventuellement substitué par un

ou plusieurs substituants identiques ou non choisis parmi les groupes hydroxyle et les atomes d'halogène (notamment fluor), de préférence un groupe alkyle en $C_1$ à $C_{12}$ linéaire ou ramifié (à l'exception du groupe tertiobutyle) ; des exemples de groupes $R_{12}$ sont les groupes méthyle, éthyle, propyle, butyle, isobutyle, hexyle, éthylhexyle, octyle, lauryle, isooctyle, isodécyle, hydroxyéthyle, hydroxypropyle, méthoxyéthyle, éthoxyéthyle et méthoxypropyle ;

  - un groupe alkyle en $C_1$ à $C_{12}$ - POE (polyoxyéthylène), avec répétition du motif oxyéthylène de 5 à 30 fois, par exemple méthoxy POE, $R_{12}$ pouvant alors être représenté par $R_{13}$-$(OC_2H_4)_n$-, où $R_{13}$ est un groupe alkyle en $C_1$ à $C_{12}$, et n est un entier variant de 5 à 30 ; ou
  - un groupement polyoxyéthylène de structure $-(CH_2CH_2O)_n-H$ où n est un entier variant de 5 à 30.

- les méthacrylates de formule :

$CH_2 = C(CH_3)COOR_{14}$

où $R_{14}$ représente :

- un groupe alkyle en C4 à C12 linéaire ou un groupe alkyle en C5 à C12 ramifié (à l'exception des groupes cycloalkyle, aryle, aralkyle, hétérocycloalkyle et hétéroaralkyle), dans lequel se trouve(nt) éventuellement intercalé(s) un ou plusieurs hétéroatomes identiques

ou non, ledit groupe alkyle pouvant en outre être éventuellement substitué par un ou plusieurs substituants identiques ou non choisis parmi les groupes hydroxyle et les atomes d'halogènes ; et de préférence un groupe alkyle en C4 à C12 linéaire ou un groupe alkyle en C5 à C12 ramifié non substitué ; des exemples de groupes R14 sont les groupes héxyle, éthylhéxyle, octyle, lauryle, isooctyle, isodécyle, dodécyle, méthoxyéthyle, méthoxypropyle et éthoxyéthyle,

- un groupe alkyle en C1 à C12 - POE (polyoxyéthylène), avec répétition du motif oxyéthylène de 5 à 30 fois, par exemple méthoxy POE, R14 pouvant alors être représenté par R13-(OC2H4)n-, où R13 est un groupe alkyle en C1 à C12, et n est un entier variant de 5 à 30 ;

ou

- un groupement polyoxyéthylène de structure -(CH2CH2O)n-H où n est un entier variant de 5 à 30.

Les monomères, particulièrement préférés, sont : l'acrylate de méthyle, l'acrylate d'éthyle, l'acrylate de propyle, l'acrylate de n-butyle, l'acrylate de 2-éthylhexyle, l'acrylate d'isobutyle, l'acrylate de méthoxyéthyle, le (méth)acrylate d'éthoxyéthyle, le (méth)acrylate de n-hexyle.

- Les esters vinyliques de formule :

$R_{15}COOCH = CH_2$

où $R_{15}$ représente un groupe alkyle en $C_2$ à $C_{12}$ linéaire ou ramifié.

Des exemples de tels esters vinyliques sont : le propionate de vinyle, le butyrate de vinyle, l'éthylhexanoate de vinyle, le néononanoate de vinyle, et le néododécanoate de vinyle.

- Les éthers d'alcool vinylique et d'alcool en $C_1$ à $C_{12}$, tels que le méthyle vinyle éther et l'éthyle vinyle éther.

Le monomère additionnel est de préférence choisi parmi les acrylates et méthacrylates décrits précédemment, et de préférence parmi les acrylates d'alkyle en $C_1$-$C_{12}$ et les méthacrylates d'alkyle en $C_4$-$C_{12}$.

Plus particulièrement, les monomères additionnels préférés sont :

- les acrylates de formule :

$CH_2 = CHCOOR_{12}$

où $R_{12}$ représente un groupe alkyle en $C_1$ à $C_{12}$ linéaire ou ramifié (à l'exception du groupe tertiobutyle), dans lequel se trouve(nt) éventuellement intercalé(s) un ou plusieurs hétéroatomes identiques ou non, ledit groupe alkyle pouvant en outre être éventuellement substitué par un ou plusieurs substituants identiques ou non choisis parmi les groupes hydroxyle et les atomes d'halogène, et

- les méthacrylates de formule :

$CH_2 = C(CH_3)COOR_{14}$

où $R_{14}$ représente un groupe alkyle en $C_4$ à $C_{12}$ linéaire ou un groupe alkyle en $C_5$ à $C_{12}$ ramifié.

**COMPOSITION**

La teneur exprimée en matière sèche en copolymère(s) présent(s) dans une composition selon la présente invention peut aller de 0,1 à 15 %, de préférence de 1 à 10 % et de façon encore plus préférée de 1 à 5 % en poids par rapport au poids total de la composition.

La composition de l'invention peut se présenter sous toutes les formes galéniques normalement utilisées pour une application topique sur la peau, notamment sous forme d'une solution aqueuse, hydroalcoolique ou huileuse, d'une émulsion huile-dans-eau, d'un gel aqueux ou huileux, d'un produit anhydre liquide, pâteux ou solide, d'une dispersion d'huile dans une phase aqueuse en présence de sphérules, ces sphérules pouvant être des nanoparticules polymériques telles que les nanosphères et les nanocapsules ou, mieux, des vésicules lipidiques de type ionique et/ou non-ionique.

Etant donné que l'on préfère formuler le copolymère tel que défini dans le cadre de la présente invention sous forme dispersée dans l'eau, on préfère les compositions comprenant une phase aqueuse.

Cette phase aqueuse peut typiquement être présente dans une teneur supérieure ou égale à 10 %, de manière préférée à 20 %, de manière encore plus préférée à 30 %, voire à 50 % en poids par rapport au poids total de la composition.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, et par exemple sous forme de stick. Elle peut être utilisée comme produit de soin et/ou comme produit de maquillage de la peau. Elle peut par exemple être utilisée comme produit du teint tel qu'un fond de teint.

La composition de l'invention constitue plus particulièrement une composition anti-âge, en particulier antirides, se présentant notamment sous forme d'un sérum, c'est-à-dire d'une composition aqueuse gélifiée. Elle peut toutefois en variante constituer une composition de soin du corps, et en particulier une composition amincissante.

De façon connue, la composition de l'invention peut contenir également les adjuvants habituels dans le domaine cosmétique, tels que les gélifiants hydrophiles ou lipophiles, les actifs hydrophiles ou lipophiles, les conservateurs, les antioxydants, les solvants, les parfums, les charges, les polymères filmogènes, les filtres, les pigments, les absorbeurs d'odeur et les matières colorantes. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, dans la phase aqueuse, dans les vésicules lipidiques et/ou dans les nanoparticules. Ces adjuvants ainsi que leurs concentrations doivent être tels qu'ils ne modifient pas la propriété recherchée de tenseur du copolymère.

Lorsque la composition de l'invention est une émulsion, la proportion de la phase grasse peut aller de 5 à 80 % en poids, et de préférence de 5 à 50 % en poids par rapport au poids total de la composition. Les matières grasses, les émulsionnants et les coémulsionnants utilisés dans la composition sous forme d'émulsion sont choisis parmi ceux classiquement utilisés dans le domaine considéré. L'émulsionnant et le coémulsionnant sont de préférence présents, dans la composition, en une proportion allant de 0,3 à 30 % en poids, et de préférence de 0,5 à 20 % en poids par rapport au poids total de la composition.

Comme matières grasses utilisables dans l'invention, on peut citer les huiles et notamment les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras (alcool cétylique), des acides gras, des cires et des gommes et en particulier les gommes de silicone.

Comme émulsionnants et coémulsionnants utilisables dans l'invention, on peut citer par exemple les esters d'acide gras et de polyéthylène glycol tels que le stéarate de PEG-50 et le stéarate de PEG-40, et les esters d'acide gras et de polyol tels que le stéarate de glycéryle et le tristéarate de sorbitane.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

En outre, on peut aussi associer aux agents tenseurs utilisés selon l'invention d'autres composés connus de l'homme du métier comme agents tenseurs et ayant des propriétés différentes de celles des agents utilisés selon l'invention, notamment les latex synthétiques, les protéines végétales, les polysaccharides d'origine végétale sous forme ou non de microgels, les amidons, les silicates mixtes et les particules colloïdales de charges inorganiques.

En variante ou en plus, lorsque la composition selon l'invention est une composition amincissante, on préfère qu'elle comprenne, dans un milieu physiologiquement acceptable, au moins un copolymère tel que défini précédemment et un ou plusieurs composés drainants, lipolytiques, désinfiltrants, amincissants, raffermissants, anti-glycants et/ou vasoprotecteurs.

La quantité d'actif(s) amincissant(s) peut varier dans une large mesure et dépend de la nature de ou des actifs utilisés. De manière générale, le ou les actifs amincissants sont présents en une concentration allant de 0,05 à 20 % et

de préférence de 0,1 à 10 % en poids par rapport au poids total de la composition.

Lorsque la composition selon l'invention constitue une composition anti-âge, il sera avantageux d'introduire dans cette composition au moins un composé choisi parmi : les agents desquamants ; les agents hydratants ; les agents dépigmentants ou propigmentants ; les agents anti-glycation ; les inhibiteurs de NO-synthase ; les agents stimulant la synthèse de macromolécules dermiques ou épidermiques et/ou empêchant leur dégradation ; les agents stimulant la prolifération des fibroblastes et/ou des kératinocytes ou stimulant la différenciation des kératinocytes ; les autres agents myorelaxants et/ou dermo-décontractants ; les agents tenseurs ; les agents anti-pollution et/ou anti-radicalaire ; les agents agissant sur la microcirculation ; les agents agissant sur le métabolisme énergétique des cellules ; et leurs mélanges.

Des exemples de tels composés additionnels sont : le rétinol et ses dérivés tels que le palmitate de rétinyle ; l'acide ascorbique et ses dérivés tels que l'ascorbyl phosphate de magnésium et le glucoside d'ascorbyle ; le tocophérol et ses dérivés tels que l'acétate de tocophéryle ; l'acide nicotinique et ses précurseurs tels que la nicotinamide ; l'ubiquinone ; le glutathion et ses précurseurs tels que l'acide L-2-oxothiazolidine-4-carboxylique ; les extraits de plantes et notamment les protéines végétales et leurs hydrolysats, ainsi que les phytohormones ; les extraits marins tels que les extraits d'algues ; les extraits bactériens ; les sapogénines telles que la diosgénine et les extraits de Wild Yam en contenant ; les céramides ; les hydroxyacides ; les hydroxyacides, tels que l'acide salicylique et l'acide n-octanoyl-5-salicylique ; le resvératrol ; les oligopeptides et pseudodipeptides et leurs dérivés acylés ; les sels de manganèse et de magnésium, en particulier les gluconates ; et leurs mélanges.

La quantité de ces actifs peut varier dans une large mesure. De manière générale, ces actifs sont présents en une concentration allant de 0,01 à 15 % et de préférence de 0,05 à 10 % en poids par rapport au poids total de la composition.

En cas d'incompatibilité, les actifs indiqués ci-dessus peuvent être incorporés dans des sphérules, notamment des vésicules ioniques ou non-ioniques et/ou des nanoparticules (nanocapsules et/ou nanosphères), de manière à les isoler les uns des autres dans la composition.

Les exemples ci-après de compositions selon l'invention sont donnés à titre d'illustration et sans caractère limitatif. Les quantités y sont données en pourcentages en poids. Les taux de neutralisation des copolymères sont de 100 %.

Dans les exemples qui suivent « Mp » est l'abréviation pour masse moléculaire au pic ; « Mn » pour masse moléculaire moyenne en nombre, « Mw » pour masse moléculaire moyenne en poids et « Ip » pour indice de polydispersité.

## Exemples

### Exemple 1 : exemple de synthèse d'un copolymère statistique méthacrylate de méthyle/acide méthacrylique

*1ère étape : Synthèse du polymère*

Dans un réacteur à double enveloppe de 21, on place 1g de Trigonox 21 S (t-butylperoxy-2-éthylhexanoate) et 200g de méthyléthyl cétone. Le mélange est chauffé au reflux pendant une heure. Après une heure, un mélange de 170g de méthacrylate de méthyle et 30g d'acide méthacrylique est ajouté au goutte à goutte sur une durée d'une heure. Le mélange incolore devient visqueux. Le chauffage est interrompu six heures après l'addition des monomères.
Composition par RMN : méthacrylate de méthyle 85,1%, acide méthacrylique 14,9 %
Masse par GPC dans le tétrahydrofurane (THF) (standards polystyrène) : Mp=98772 $g.mol^{-1}$ ; Mn = 61261 $g.mol^{-1}$ ; Mw= 105698 $g.mol^{-1}$ Ip=1,7

*2ème étape : Mise en dispersion du polymère dans l'eau*

Au milieu réactionnel ci-dessus, on rajoute 200g de méthyléthyl cétone et on chauffe à 60 °C. On ajoute goutte à goutte 30,86 g d'amino-2-méthyl-2-propanol pour neutraliser les fonctions acides, et 1200 g d'eau. On évapore les solvants volatils en chauffant jusqu'à 100°. On obtient une dispersion aqueuse jaune transparente.

### Exemple 2 : exemple de synthèse d'un copolymère statistique méthacrylate de cyclohexyle/acide méthacrylique

*1ère étape : Synthèse du polymère*

Dans un tricol équipé d'un barreau aimanté, on introduit 200 g de méthyléthyl cétone, puis les monomères, 42,5 g de méthacrylate de cyclohexyle et 7,5 g d'acide méthacrylique. On fait circuler un courant d'azote dans le ballon pendant quelques minutes de manière à éliminer l'oxygène ambiant. Enfin on introduit l'amorceur, 155 µL de trigonox

21 S (t-butylperoxy-2-éthylhexanoate). Le milieu réactionnel est ensuite chauffé dans un bain d'huile à 80 °C pendant 8 heures. On obtient un produit visqueux.

On précipite le polymère en ajoutant goutte à goutte le milieu réactionnel dans 4 1 de méthanol sous agitation. Le polymère est récupéré par filtration puis séché sous vide. Rendement 83 %
Composition par RMN : méthacrylate de cyclohexyle 83,7 %, acide méthacrylique 16,3 %
Masse par GPC dans le THF (standards polystyrène) : Mp=134315 g.mol$^{-1}$ ; Mn = 85905 g.mol$^{-1}$ ; Mw= 159093 g.mol$^{-1}$
Ip=1,85

*2ème étape : Mise en dispersion du polymère dans l'eau*

On dissout 40 g du polymère ci-dessus dans 220 g de tétrahydrofurane. On neutralise les fonctions acides en introduisant 6,75 g d'amino-2-méthyl-2-propanol. 160 g d'eau sont ensuite ajoutés au milieu puis on évapore le tétrahydrofurane sous pression réduite à l'évaporateur rotatif. Après évaporation, on obtient une dispersion opalescente à 22 % massique dans l'eau.

<u>Exemple 3 : exemple de synthèse d'un copolymère statistique méthacrylate de méthyle/acrylate de méthyle/acide méthacrylique</u>

*1ère étape : Synthèse du polymère*

Dans un tricol équipé d'un barreau aimanté, on introduit 50 g de méthyléthyl cétone, puis les monomères, 35 g de méthacrylate de méthyle, 7,5 g d'acrylate de méthyle et 7,5 g d'acide méthacrylique. On fait circuler un courant d'azote dans le ballon pendant quelques minutes de manière à éliminer l'oxygène ambiant. Enfin on introduit l'amorceur, 143 µL de trigonox 21S (t-butylperoxy-2-éthylhexanoate). Le milieu réactionnel est ensuite chauffé dans un bain d'huile à 80 °C pendant 8 heures. On obtient un produit visqueux.

On précipite le polymère en ajoutant goutte à goutte le milieu réactionnel dans 21 d'un mélange éthanol/eau (50/50) sous agitation. Le polymère est récupéré par filtration puis séché sous vide. Rendement 54 %
Composition par RMN : méthacrylate de méthyle 71 %, acrylate de méthyle 14 % ; acide méthacrylique 16 %
Masse par GPC dans le THF (standards polystyrène) : Mp=113249 g.mol$^{-1}$ ; Mn = 71648 g.mol$^{-1}$ ; Mw= 105358 g.mol$^{-1}$
Ip=1,47

*2ème étape : Mise en dispersion du polymère dans l'eau*

On dissout 26,8 g du polymère ci-dessus dans 110 g de tétrahydrofurane et 10g d'EtOH. On chauffe à 60 °C pendant 6h pour solubiliser l'ensemble puis on laisse revenir à température ambiante. On neutralise les fonctions acides en introduisant 4,16 g d'amino-2-méthyl-2-propanol. 150 g d'eau sont ensuite ajoutés au milieu puis on évapore le tétrahydrofurane et l'éthanol sous pression réduite à l'évaporateur rotatif. Après évaporation, on obtient un gel opalescent cohésif à 12,5 % massique dans l'eau.

<u>Exemple 4 : exemple de synthèse d'un copolymère statistique méthacrylate de méthyle / acrylate d'éthyle / acide méthacrylique</u>

*1 ère étape : Synthèse du polymère*

Dans un tricol équipé d'un barreau aimanté, on introduit 50 g de méthyléthyl cétone, puis les monomères, 37,5 g de méthacrylate de méthyle, 5 g d'acrylate d'éthyle et 7,5 g d'acide méthacrylique. On fait circuler un courant d'azote dans le ballon pendant quelques minutes de manière à éliminer l'oxygène ambiant. Enfin on introduit l'amorceur, 143 µL de trigonox 21S (t-butylperoxy-2-éthylhexanoate). Le milieu réactionnel est ensuite chauffé dans un bain d'huile à 80 °C pendant 8 heures. On obtient un produit visqueux.

On précipite le polymère en ajoutant goutte à goutte le milieu réactionnel dans 21 d'un mélange éthanol/eau (50/50) sous agitation. Le polymère est récupéré par filtration puis séché sous vide. Rendement 92 %
Composition par RMN : méthacrylate de méthyle 75,7 %, acrylate d'éthyle 6,7 %; acide méthacrylique 15 %
Masse par GPC dans le THF (standards polystyrène) : Mp=115523 g.mol$^{-1}$; Mn = 63149 g.mol$^{-1}$ ; Mw= 124703 g.mol$^{-1}$
Ip= 1,97

*2ème étape : Mise en dispersion du polymère dans l'eau*

On dissout 37,5 g du polymère ci-dessus dans 150 g de tétrahydrofurane et 10 g d'EtOH. On chauffe à 60 °C

pendant 6h pour solubiliser l'ensemble puis on laisse revenir à température ambiante. On neutralise les fonctions acides en introduisant 5,8 g d'amino-2-méthyl-2-propanol. 210 g d'eau sont ensuite ajoutés au milieu puis on évapore le tétrahydrofurane et l'éthanol sous pression réduite à l'évaporateur rotatif. Après évaporation, on obtient dispersion transparente visqueuse à 13,6 % massique dans l'eau.

Exemple 5 : exemple de synthèse d'un copolymère statistique méthacrylate de méthyle / acide méthacrylique

*1ère étape : Synthèse du polymère*

Dans un réacteur à double enveloppe de 21, on place 4,8 g de Trigonox 21 S (t-butylperoxy-2-éthylhexanoate) et 360 g de méthyléthyl cétone. Le mélange est chauffé au reflux pendant une heure. Après une heure, un mélange de 102 g de méthacrylate de méthyle et 18 g d'acide méthacrylique est ajouté au goutte à goutte sur une durée d'une heure. Le mélange incolore devient visqueux. Le chauffage est interrompu six heures après l'addition des monomères.
Composition par RMN : méthacrylate de méthyle 86,7 %, acide méthacrylique 13,3 %
Masse par GPC dans le tétrahydrofurane (THF) (standards polystyrène) : Mp=25200 $g.mol^{-1}$ ; Mn = 13900 $g.mol^{-1}$ ; Mw= 30450$g.mol^{-1}$ Ip=2,19

*2ème étape : Mise en dispersion du polymère dans l'eau*

Au milieu réactionnel ci-dessus, on ajoute goutte à goutte et à température ambiante 16,5 g d'amino-2-méthyl-2-propanol pour neutraliser les fonctions acides, et 360 g d'eau. On évapore les solvants volatils en chauffant jusqu'à 100°. On obtient une dispersion aqueuse jaune transparente d'extrait sec 26,9 %

Exemple 6 (comparatif) : exemple de synthèse d'un copolymère statistique méthacrylate de méthyle/acide méthacrylique

*1ère étape : Synthèse du polymère*

Dans un tricol équipé d'un barreau aimanté, on introduit 300 g d'éthanol, puis les monomères, 50 g de méthacrylate de méthyle et 50 g d'acide méthacrylique. On fait circuler un courant d'azote dans le ballon pendant quelques minutes de manière à éliminer l'oxygène ambiant. Enfin on introduit l'amorceur, 0,5 g de trigonox 21S (t-butylperoxy-2-éthylhexanoate). Le milieu réactionnel est ensuite chauffé dans un bain d'huile à 90 °C pendant 8 heures. On obtient un produit visqueux.

On dilue le milieu réactionnel avec 150 g d'éthanol et on précipite en ajoutant goutte à goutte dans 2 1 d'eau sous agitation. Le polymère est récupéré par filtration puis séché sous vide. Le rendement s'élève à 80 %.
Composition par RMN : méthacrylate de méthyle 65,6 %, acide méthacrylique 34,4 %

*2ème étape : Mise en dispersion du polymère dans l'eau*

On dissout 50,6 g du polymère ci-dessus dans 204 g de Tétrahydrofurane. On neutralise les fonctions acides en introduisant 18 g d'amino-2-méthyl-2-propanol. 184 g d'eau sont ensuite ajoutés au milieu puis on évapore le tétrahydrofurane sous pression réduite à l'évaporateur rotatif. Après évaporation, on obtient une dispersion opaque à 17% massique dans l'eau.

Exemple 7 (comparatif) : exemple de synthèse d'un copolymère statistique méthacrylate de méthyle / acide méthacrylique

*1ère étape : Synthèse du polymère*

Dans un réacteur à double enveloppe de 21, on place 8 g de Trigonox 21 S (t-butylperoxy-2-éthylhexanoate) et 300 g de méthyléthyl cétone. Le mélange est chauffé au reflux pendant une heure. Après une heure, un mélange de 85 g de méthacrylate de méthyle et 15 g d'acide méthacrylique est ajouté au goutte à goutte sur une durée d'une heure. Le mélange incolore devient visqueux. Le chauffage est interrompu six heures après l'addition des monomères.
Composition par RMN : méthacrylate de méthyle 80,3 %, acide méthacrylique 19,7%
Masse par GPC dans le tétrahydrofurane (THF) (standards polystyrène) : Mp=11100 $g.mol^{-1}$ ; Mn = 6600 $g.mol^{-1}$ ; Mw= 14600$g.mol^{-1}$ Ip=2,21

*2ème étape : Mise en dispersion du polymère dans l'eau*

Au milieu réactionnel ci-dessus, on ajoute goutte à goutte et à température ambiante 20,4 g d'amino-2-méthyl-2-propanol pour neutraliser les fonctions acides, et 300 g d'eau. On évapore les solvants volatils en chauffant jusqu'à 100°. On obtient une dispersion aqueuse jaune transparente d'extrait sec 29,6 %.

Exemple 8 : sérum antirides

On a préparé la composition suivante :

A

- Eau 50,45 g
- Polymère épaississant synthétique 2,00 g
- Conservateurs 0,85 g

B

- Copolymère de l'exemple 1 (dispersion à 15 % dans l'eau) 46,70 g

*Mode opératoire:*

On chauffe à 75 °C environ, sous agitation, la phase A, on arrête le chauffage tout en maintenant l'agitation jusqu'au retour à température ambiante et l'on ajoute alors la phase B. Une légère agitation est ensuite maintenue pendant 30 minutes.

Exemple 9 : crème antirides (émulsion H/E)

On a préparé la composition suivante :

A

- Glycéryl stéarate (et) PEG-100 stéarate 2,00 g
- Dimyristyl tartrate (et) cétéaryl alcohol (et) C12-15 pareth-7 (et) PPG-25 laureth-25 1,50 g
- Cyclohexasiloxane 10,00 g
- Alcool stéarylique 1,00 g

B

- Eau 37,15 g
- Phénoxyéthanol 1,00 g
- Pentasodium éthylène diamine tetraméthylène phosphate 0,05 g
- Ammonium Polyacryldimethyltauramide (épaississant) 0,40 g
- Gomme de xanthane 0,20 g

C

- Copolymère de l'exemple 1 (dispersion à 15 % dans l'eau) 46,70 g

*Mode opératoire:*

On chauffe les constituants de la phase B excepté l'épaississant à environ 75 °C et on y incorpore ensuite l'épaississant ; on agite jusqu'à obtention d'un gel homogène.
On chauffe la phase A à environ 75 °C, puis on réalise l'émulsion en incorporant la phase A dans la phase B.
Enfin, à 40-45 °C, on incorpore la phase C et on maintient l'agitation jusqu'à refroidissement complet.
Le sérum appliqué sur le visage permet d'estomper les rides.
Des compositions similaires ont également été préparées en utilisant respectivement le polymère des exemples

3, 4 et 5. Les compositions obtenues appliquées sur le visage permettent de lisser efficacement les rides.

Exemple 10: Mise en évidence de l'effet tenseur des polymères selon l'invention

10-1 Quantification *in vitro* de l'effet tenseur des copolymères

Il a été montré que l'effet tenseur pouvait être décrit par un test *in vitro* de rétraction. Cet essai consiste à quantifier *in vitro* le pouvoir tenseur d'un matériau déposé sur un substrat en élastomère ayant un module de l'ordre de 20 MPa et d'une épaisseur de 100 μm. La solution contenant l'agent tenseur à 7 % en poids est donc déposée (30 μl) sur une éprouvette rectangulaire (10x40mm) d'élastomère. Après 3-4 heures de séchage à 22±3 °C et 40±10 % HR, la tension exercée par ce dépôt sur le substrat et par conséquent le potentiel tenseur est directement relié à la diminution de la largeur au centre de l'éprouvette. L'effet tenseur (ET) peut alors se quantifier de la façon suivante :

$$\text{« ET »} = (L_0 - L_{3h} / L_0)\text{x}100 \text{ en } \%$$

avec $L_o$ = largeur initiale 10mm
et $L_{3h}$ = largeur après 3 heures de séchage

Le résultat présenté ci-dessous montre au regard de l'application cosmétique recherchée un effet tenseur très important obtenu avec les copolymères conformes à l'invention et ce, même en présence de glycérine.

| | ET(%) |
|---|---|
| copolymère de l'exemple 1(7 % dans $H_2O$) | 83±16 |
| copolymère de l'exemple 1 (7 % dans $H_2O$) + 3 % glycérine | 65±15 |
| copolymère de l'exemple 2 (7 % dans $H_2O$) | 80±8 |
| copolymère de l'exemple 2 (7 % dans $H_2O$) + 3 % glycérine | 85±12 |
| copolymère de l'exemple 3 (7 % dans $H_2O$) | 100±5 |
| copolymère de l'exemple 4 (7 % dans $H_2O$) | 95±9 |
| copolymère de l'exemple 5 (7 % dans $H_2O$) | 71±4 |
| copolymère de l'exemple 6 comparatif (7% dans $H_2O$) | 15±6 |
| copolymère de l'exemple 6 comparatif (7 % dans $H_2O$) + 3 % glycérine | 0 |
| copolymère de l'exemple 7 comparatif (7 % dans $H_2O$) | 29±3 |

10-2 Evaluation *in vivo* sur femmes à peaux matures (test cabine) de l'effet anti-rides des compositions cosmétiques contenant le copolymère de l'exemple 1:

La composition correspondant à l'exemple 8 a été testée sur un panel de 6 femmes âgées de 40 à 60 ans présentant des rides et ridules au niveau du contour de l'oeil. Après application de ces formulations il a été observé sur tous les modèles un effet important de lissage mécanique des rides et des ridules.

10-3 Détermination des températures de transition vitreuse des copolymères :

Les températures de transition vitreuse ont été déterminées par DSC selon le protocole rapporté dans la description.

| | Tg (°C) |
|---|---|
| copolymère de l'exemple 1 | 81 °C |
| copolymère de l'exemple 2 | 88 °C |
| copolymère de l'exemple 6 comparatif | 40 °C |

Les températures de transition vitreuse des copolymères des exemples 3, 4, 5 et 7 sont supérieures à 45 °C.

## Revendications

1. Composition cosmétique comprenant, dans un milieu physiologiquement acceptable, au moins un copolymère statistique à chaîne principale linéaire de nature éthylénique, dans laquelle ledit copolymère :

   (i) présente une masse moléculaire en poids comprise entre 15 000 et 600 000 g/mol, et consiste en :
   (ii) au moins 70 % en poids d' « unités monomères » issues de monomères dont les homopolymères sont hydrophobes et présentent une température de transition vitreuse supérieure à 40 °C,
   (iii) au moins une « unité monomère » issue d'un monomère hydrophile ionique au moins partiellement neutralisée, et
   (iv) 0 à 25 % en poids d'« unités monomères » issues de monomères additionnels dont les homopolymères présentent une température de transition vitreuse inférieure à 40 °C choisis parmi :

   - les acrylates de formule :

     $$CH_2 = CHCOOR_{12}$$

     où $R_{12}$ représente un groupe alkyle en $C_1$ à $C_{12}$ linéaire ou ramifié à l'exception du groupe tertiobutyle, dans lequel se trouve(nt) éventuellement intercalé(s) un ou plusieurs hétéroatomes identiques ou non, ledit groupe alkyle pouvant en outre être éventuellement substitué par un ou plusieurs substituants identiques ou non choisis parmi les groupes hydroxyle et les atomes d'halogène, et
   - les méthacrylates de formule :

     $$CH_2 = C(CH_3)COOR_{14}$$

   où $R_{14}$ représente un groupe alkyle en $C_4$ à $C_{12}$ linéaire ou un groupe alkyle en $C_5$ à $C_{12}$ ramifié, et
   (v) présente une température globale de transition vitreuse supérieure ou égale à 45 °C.

2. Composition selon la revendication 1, dans laquelle le copolymère est en dispersion dans l'eau.

3. Composition selon l'une quelconque des revendications 1 à 2, dans laquelle le copolymère comprend plus de 75 %, de préférence plus de 80 % en poids par rapport à son poids total d'« unités monomères » issues de monomères dont les homopolymères présentent une température de transition vitreuse supérieure à 40 °C, et jusqu'à 95 % en poids.

4. Composition selon l'une quelconque des revendications 1 à 3, dans laquelle les monomères dont les homopolymères présentent une température de transition vitreuse supérieure à 40 °C sont choisis parmi les composés vinyliques, les acrylates, les méthacrylates et les (méth)acrylamides.

5. Composition selon l'une quelconque des revendications 1 à 4, dans laquelle les monomères dont les homopolymères présentent une température de transition vitreuse supérieure à 40 °C sont choisis parmi :

   - les composés vinyliques de formule :

     $$CH_2 = CHR_1,$$

   où $R_1$ est un groupe

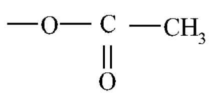

,

un groupe cycloalkyle en $C_3$ à $C_8$ ou un groupe aryle en $C_6$ à $C_{20}$,
- les acrylates de formule :

$$CH_2 = CHCOOR_2$$

où $R_2$ est un groupe tertiobutyle, un groupe cycloalkyle en $C_3$ à $C_8$ éventuellement ponté par un groupe alkylène en $C_1$ à $C_4$, ou un groupe aralkyle en $C_7$ à $C_{30}$ (groupe alkyle en $C_1$ à $C_4$),
- les méthacrylates de formule :

$$CH_2 = C(CH_3)\ COOR_3$$

où $R_3$ est un groupe isobutyle, tertiobutyle, un groupe alkyle en $C_1$ à $C_3$ linéaire ou ramifié, un groupe cycloalkyle en $C_3$ à $C_8$ éventuellement ponté par un groupe alkylène en $C_1$ à $C_4$ ou un groupe aralkyle en $C_7$ à $C_{30}$ (groupe alkyle en $C_1$ à $C_4$), et
- les (méth) acrylamides de formule :

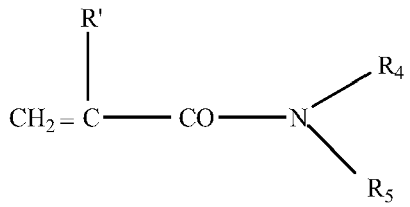

où R' désigne H ou $-CH_3$, et où $R_4$ et $R_5$ identiques ou différents représentent chacun un atome d'hydrogène ou un groupe alkyle linéaire ou ramifié en $C_4$ à $C_{12}$, étant entendu que $R_4$ et $R_5$ ne peuvent représenter simultanément un atome d'hydrogène.

**6.** Composition selon l'une quelconque des revendications 1 à 5, dans laquelle les monomères dont les homopolymères présentent une température de transition vitreuse supérieure à 40 °C sont choisis parmi le styrène, l'acrylate de benzyle, l'acrylate de cycloalkyle en $C_3$ à $C_8$ éventuellement ponté par un groupe alkylène en $C_1$ à $C_4$, l'acrylate de tertiobutyle, le méthacrylate de ($C_1$-$C_3$)alkyle, le méthacrylate de tertiobutyle, le méthacrylate de benzyle et le méthacrylate de cycloalkyle en $C_3$ à $C_8$ éventuellement ponté par un groupe alkylène en $C_1$ à $C_4$.

**7.** Composition selon l'une quelconque des revendications 1 à 6, dans laquelle les monomères dont les homopolymères présentent une température de transition vitreuse supérieure à 40 °C sont choisis parmi le vinylcyclohexane, le styrène, l'acétate de vinyle, l'acrylate de benzyle, l'acrylate de cyclohexyle, l'acrylate de tertiobutyle, l'acrylate d'isobornyle et l'acrylate de norbornyle, le méthacrylate de méthyle, d'éthyle, d'isobutyle, de cyclohexyle, de benzyle, de tertiobutyle, d'isobornyle et de norbornyle, le N-butylacrylamide, le N-t-butylacrylamide et le N,N-dibutylacrylamide.

**8.** Composition selon l'une quelconque des revendications 1 à 7, dans laquelle les monomères dont les homopolymères présentent une température de transition vitreuse supérieure à 40 °C sont choisis parmi l'acrylate de benzyle, l'acrylate de cyclohexyle, l'acrylate de tertiobutyle, l'acrylate d'isobornyle et l'acrylate de norbornyle, le méthacrylate de méthyle, d'éthyle, d'isobutyle, de cyclohexyle, de benzyle, de tertiobutyle, d'isobornyle et de norbornyle et le styrène.

**9.** Composition selon l'une quelconque des revendications précédentes, dans laquelle les monomères dont les homopolymères présentent une température de transition vitreuse supérieure à 40 °C sont choisis parmi le méthacrylate de méthyle et le méthacrylate de cyclohexyle.

**10.** Composition selon l'une quelconque des revendications 1 à 9, dans laquelle le monomère hydrophile ionique est choisi parmi les monomères hydrophiles anioniques, les monomères hydrophiles cationiques, les monomères amphotères et leurs mélanges.

**11.** Composition selon la revendication 10, dans laquelle le monomère hydrophile anionique est défini par la formule (I) :

$$CH_2=CR_6\ (Z)_n\ (R_7)_m\ Y \qquad (I)$$

où Z prend l'une des significations suivantes : C(=O)O, C(=O)NH, O, O(C=O),
n est égal à 0 ou 1,
m est égal à 0 ou 1,
$R_6$ est un atome d'hydrogène, un groupe $CH_3$, ou un groupe ($C_2$-$C_{30}$)akyle,
$R_7$ est choisi parmi les groupements alkylènes linéaires saturés ou non et/ou branchés et/ou cycliques (aromatiques ou non) en $C_1$ à $C_{30}$, incluant ou non un ou plusieurs hétéroatomes, et
Y est choisi parmi les groupes suivants : -COOH, $-SO_3H$, $-OSO_3H$, $-OP(OH)_2$, $-OPO(OH)_2$.

**12.** Composition selon la revendication 11, dans laquelle n est égal à 0 dans la formule (I).

**13.** Composition selon la revendication 11 ou 12, dans laquelle m est égal à 0 dans la formule (I).

**14.** Composition selon l'une quelconque des revendications 11 à 13, dans laquelle $R_6$ est un atome d'hydrogène, un groupe $CH_3$ ou un $C_2H_5$ et $R_7$ est choisi parmi les groupes alkylène en $C_1$ à $C_{30}$, phénylène, benzylène, $-(CH_2-CH=CH)-$ et (CHOH).

**15.** Composition selon la revendication 14, dans laquelle $R_6$ est un groupe $CH_3$.

**16.** Composition selon l'une quelconque des revendications 10 à 15, dans laquelle le monomère hydrophile anionique est choisi parmi :

- les monomères à insaturation(s) éthylénique(s) comprenant au moins une fonction acide carboxylique (COOH), phosphonique ($PO_3H_2$) ou sulfonique ($SO_3H$), comme par exemple l'acide acrylique, l'acide méthacrylique, le (méth)acrylate de 2-carboxyéthyle, l'acide vinylbenzoïque, l'acide acrylamidoglycolique $CH_2=CHCONHCH(OH)CO_2H$, l'acide maléique, l'acide acrylamidopropanesulfonique, l'acide acrylamido 2-méthylpropanesulfonique, l'acide styrène sulfonique, l'acide vinylsulfonique, le méthacrylate ou l'acrylate d'acide propyl-3-sulfonique ($CH_2=C(CH_3)CO_2(CH_2)_3SO_3H$), le méthacrylate ou l'acrylate d'acide éthyl-2-sulfonique ($CH_2=C(CH_3)CO_2(CH_2)_2SO_3H$) et la vinylméthylsulfone, l'acide vinylphosphonique ($CH_2=CH-PO(OH)_2$), le méthacrylate d'acide éthyl-2-phosphonique ($CH_2=C(CH_3)COOCH_2CH_2OP(O)(OH)_2$),
- les anhydrides carboxyliques porteurs d'une liaison vinylique tels que l'anhydride maléique,
- les diacides tels que l'acide crotonique, l'acide itaconique, l'acide fumarique, l'acide maléique,
- ainsi que leurs mélanges.

**17.** Composition selon l'une quelconque des revendications précédentes, dans laquelle le monomère hydrophile est l'acide (méth)acrylique.

**18.** Composition selon l'une quelconque des revendications 1 à 17, dans laquelle la teneur en poids d'« unités monomères » issues de monomères hydrophiles ioniques dans le copolymère est comprise entre 5 et 30 % préférentiellement entre 10 et 25 % et de façon encore plus préférée entre 10 et 20 % par rapport au poids total du copolymère.

**19.** Composition selon l'une quelconque des revendications 1 à 18, dans laquelle le copolymère est choisi parmi

- Les copolymères de méthacrylate de méthyle / acide méthacrylique ; les copolymères de méthacrylate de méthyle / acide acrylique, lesdits copolymères contenant entre 70 et 90 % en poids de méthacrylate de méthyle ;
- Les copolymères de méthacrylate d'éthyle/ acide méthacrylique ; les copolymères de méthacrylate d'éthyle / acide acrylique, lesdits copolymères contenant entre 70 et 90 % en poids de méthacrylate d'éthyle ;
- Les copolymères de méthacrylate d'isobutyle/ acide méthacrylique ; les copolymères de méthacrylate d'isobutyle / acide acrylique, lesdits copolymères contenant entre 70 et 90 % en poids de méthacrylate d'isobutyle ;
- Les copolymères de méthacrylate de benzyle / acide méthacrylique ; les copolymères de méthacrylate de benzyle / acide acrylique, lesdits copolymères contenant entre 70 et 90 % en poids de méthacrylate de benzyle ;
- Les copolymères d'acrylate de benzyle / acide méthacrylique ; les copolymères d'acrylate de benzyle / acide acrylique, lesdits copolymères contenant entre 70 et 90 % en poids d'acrylate de benzyle ;
- Les copolymères de méthacrylate de cyclohexyle / acide méthacrylique ; les copolymères de méthacrylate de cyclohexyle / acide acrylique, lesdits copolymères contenant entre 70 et 90 % en poids de méthacrylate de cyclohexyle ;

- Les copolymères d'acrylate de cyclohexyle / acide méthacrylique ; les copolymères d'acrylate de cyclohexyle / acide acrylique, lesdits copolymères contenant entre 70 et 90 % en poids d'acrylate de cyclohexyle ;
- Les copolymères de méthacrylate de tertio-butyle/ acide méthacrylique ; les copolymères de méthacrylate de tertio-butyle / acide acrylique, lesdits copolymères contenant entre 70 et 90 % en poids de méthacrylate de tertio-butyle ;
- Les copolymères d'acrylate de tertio-butyle/ acide méthacrylique ; les copolymères d'acrylate de tertio-butyle / acide acrylique, lesdits copolymères contenant entre 70 et 90 % en poids d'acrylate de tertio-butyle ;
- Les copolymères de méthacrylate d'isobornyle/ acide méthacrylique ; les copolymères de méthacrylate d'isobornyle / acide acrylique, lesdits copolymères contenant entre 70 et 90 % en poids de méthacrylate d'isobornyle ;
- Les copolymères d'acrylate d'isobornyle / acide méthacrylique ; les copolymères d'acrylate d'isobornyle / acide acrylique, lesdits copolymères contenant entre 70 et 90 % en poids d'acrylate d'isobornyle ;
- Les copolymères de méthacrylate de norbornyle/ acide méthacrylique ; les copolymères de méthacrylate de norbornyle / acide acrylique, lesdits copolymères contenant entre 70 et 90 % en poids de méthacrylate de norbornyle ;
- Les copolymères d'acrylate de norbornyle / acide méthacrylique ; les copolymères d'acrylate de norbornyle / acide acrylique, lesdits copolymères contenant entre 70 et 90 % en poids d'acrylate de norbornyle et ;
- Les copolymères de styrène / acide méthacrylique ; les copolymères de styrène / acide acrylique, lesdits copolymères contenant entre 70 et 90 % en poids de styrène.

**20.** Composition selon l'une quelconque des revendications 1 à 19, dans laquelle le copolymère comprend en outre au moins une « unité monomère » issue d'un monomère dont l'homopolymère présente une température de transition vitreuse inférieure à 40 °C.

**21.** Composition selon la revendication 20, dans laquelle le monomère dont l'homopolymère présente une température de transition vitreuse inférieure à 40 °C est présent en une teneur inférieure ou égale à 25 %, de préférence de 5 % à 25 % en poids par rapport au poids total du copolymère, et de préférence en une teneur inférieure ou égale à 10 % en poids, de préférence de 5 à 10 % en poids.

**22.** Composition selon l'une quelconque des revendications 1 à 21, dans laquelle le copolymère tel que défini dans l'une quelconque des revendications 1 à 20 est compris dans une teneur exprimée en matière sèche allant de 0,1 à 15 %, de préférence de 1 à 10 % et de façon encore plus préférée de 1 à 5 % en poids par rapport au poids total de la composition.

**23.** Utilisation comme agent tenseur, dans une composition cosmétique, d'au moins un copolymère tel que défini selon l'une quelconque des revendications 1 à 21.

**24.** Procédé de traitement cosmétique d'une peau vieillie, notamment ridée, comprenant l'application sur ladite peau d'au moins une composition selon l'une quelconque des revendications 1 à 22, en une quantité efficace pour estomper les rides par un effet tenseur.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9829091 A
- WO 9619180 A
- WO 9829092 A
- EP 1038519 A
- WO 0030595 A
- FR 2843025
- FR 2822676